(19) Europäisches Patentamt
European Patent Office
Office européen des brevets

(11) EP 1 645 557 A1

# (12) EUROPEAN PATENT APPLICATION

published in accordance with Art. 158(3) EPC

(43) Date of publication:
**12.04.2006 Bulletin 2006/15**

(21) Application number: **04729237.0**

(22) Date of filing: **23.04.2004**

(51) Int Cl.:
***C07D 333/46** (1985.01)*
***A61K 36/00** (0000.00)*
***A61P 3/10** (2000.01)*
***A23L 1/30** (1968.09)*
***A61K 31/381** (2000.01)*
***A61P 3/04** (2000.01)*
***A61P 43/00** (2000.01)*
***C12N 9/99** (1980.01)*

(86) International application number:
**PCT/JP2004/005865**

(87) International publication number:
**WO 2004/094402 (04.11.2004 Gazette 2004/45)**

(84) Designated Contracting States:
**DE FR GB IT**

(30) Priority: **24.04.2003 JP 2003120317**

(71) Applicant: **Morishita Jintan Co., Ltd.**
**Osaka 540-8566 (JP)**

(72) Inventors:
- **ASADA, Masanori,**
  **c/o Morishita Jintan Co., Ltd.**
  **Osaka-shi,**
  **Osaka 5408566 (JP)**
- **KAWAHARA, Yuzo,**
  **c/o Morishita Jintan Co., Ltd.**
  **Osaka-shi,**
  **Osaka 5408566 (JP)**
- **KITAMURA, Shinichi**
  **Osaka 5998125 (JP)**

(74) Representative: **von Kreisler, Alek et al**
**Deichmannhaus am Dom,**
**Postfach 10 22 41**
**50462 Köln (DE)**

## (54) NOVEL SUBSTANCE HAVING ALPHA-GLUCOSIDASE INHIBITING ACTIVITY AND FOOD CONTAINING THE SAME

(57) [PROBLEMS]

To provide a novel substance having α-glucosidase inhibiting activity, derived from natural products and being safe, which substance has not only a blood sugar ameliorating activity being effective in the prevention or treatment of diabetes or obesity but also an activity of reducing postprandial hyper-blood sugar, etc. and to provide food containing the same.

[MEANS FOR SOLVING PROBLEMS]

A substance having α-glucosidase inhibiting activity, represented by the formula:

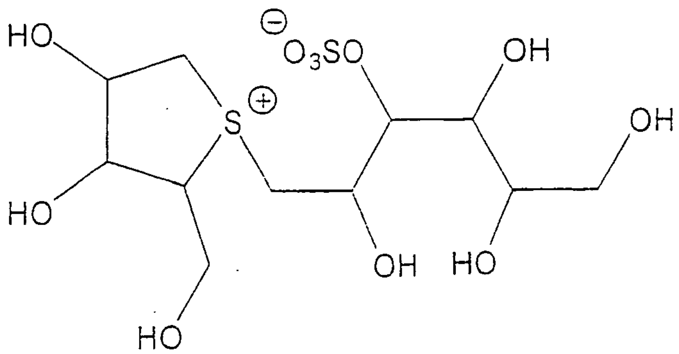

EP 1 645 557 A1

## Description

Technical Field

The present invention relates to a substance having α-glucosidase inhibiting activity effective in prevention or treatment of diabetes or obesity, and in particular to a novel substance having α-glucosidase inhibiting activity, which is contained in solvent extracts from plants of the family Hippocrateaceae or the family Celastraceae; the genus Salacia.

Background Art

Number of patients with diabetes, one of life style-related diseases, is increasing at present, and is said to reach three hundred million in the world in the year 2025. Diabetes is roughly divided into type I diabetes (insulin dependent diabetes) attributable mainly to reduction or disappearance in the ability to secrete insulin and type II diabetes (insulin non-dependent diabetes) caused by the reduction in insulin susceptibility due to obesity etc. A majority of patients with diabetes in Japan have type II diabetes that is often accompanied by obesity.
The control of glucose levels in blood is necessary and essential for treatment of type II diabetes. Accordingly, it is very important that digestion of ingested carbohydrates is suppressed thereby suppressing an excessive increase in glucose levels in blood after a meal.
Men who even if not developing diabetes, have obesity or relatively high blood sugar levels attributable mainly to ingestion of high-calorie food in Westernized eating habits, to overeating, and to lack of exercise are also increasing. Such men are regarded as those with potential diabetes, and are spreading to children at present. The suppression of an excessive increase in postprandial glucose levels in blood as described above is considered as an important approach not only to treatment of patients with diabetes but also to amelioration of obesity in those with potential diabetes to prevent diabetes.
As one of digestion enzymes possessed by humans, there is α-glucosidase. In a biochemical process of biological polymers, this enzyme plays an important function in degrading disaccharides such as sucrose and maltose into monosaccharides (for example, glucose) easily absorbed into the living body The suppression of an excessive increase in postprandial sugar levels, as a result of the reduction in the amount of sugars absorbed into the living body by inhibiting or regulating the function of α-glucosidase, is considered to be an effective means not only for prevention and treatment of type II diabetes but also for amelioration of diabetes and prevention of diabetes in those with potential diabetes.
As α-glucosidase inhibitors developed for the purpose of inhibiting the function of α-glucosidase, two medicines, that is, Basen® (Takeda Chemical Industries, Ltd.) and Glucobay® (Bayer Yakuhin, Ltd.) are approved. These medicines account for about 15% of diabetic medicines in the market. The α-glucosidase inhibitors exhibit not only an action as a diabetic remedy but also an effect on amelioration of obesity due to overeating or lack of exercise and also on dieting. However, it is reported that, though in rare cases, these α-glucosidase inhibitors cause severe side effects such as a hepatic functional disorder and low blood sugar. Accordingly, these α-glucosidase inhibitors should be strictly formulated under the supervision of a physician, and cannot be easily obtained or digested under the present situation.
Substances derived from natural products and having α-glucosidase inhibiting activity attract attention because they are safer with relatively mild side effects in the living body than the synthetic pharmaceutical preparations described above. Examples of such substances include deoxyrizinomycin contained in a mulberry (Morus bombycis Koidzumi) and a dayflower (Commehna communis) [Natural Medicines, 55(5), 251-254 (2001)], Salacinol contained in Salacia reticulata, the genus Salacia, the family Celastraceae (Yoshikawa M., Tetrahedron Lett., 38, 8367-8370 (1997)), and kotalanol (Yoshikawa M., Chem. Pharm. Bull., 46(8), 1339-1340 (1998)). In particular, Salacia reticulata is considered to contain a substance exhibiting a particularly high α-glucosidase inhibiting activity.
Salacia reticulata is a climbing perennial woody plant growing wild mainly in the southern part in India and the northern part in Sri Lanka. Its roots and stems have been used since old times in ayurveda that is traditional medicine in South Asia, in order to relieve a subjective symptom (dry mouth), so Salacinol and kotalanol contained therein are considered to be relatively safe substances in the living body. The above-mentioned Salacinol and kotalanol are thioglycosulfonyl intramolecular sulfate structures represented by the following formulae, respectively. The α-glucosidase inhibiting activity of the two is reported to be almost equivalent to that of a presently marketed synthetic medicine Glucobay® (Bayer Yakuhin, Ltd.) (Yoshikawa M., Chem. Phar. Bull., 46(8), 1339-1340 (1998)).

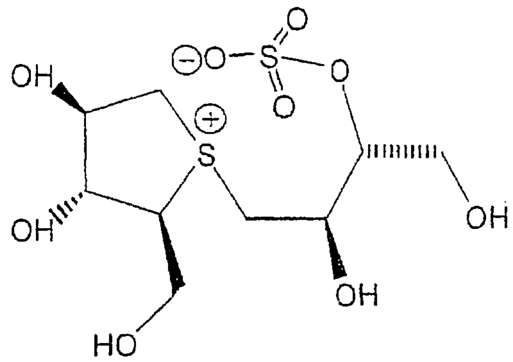

Salacinol

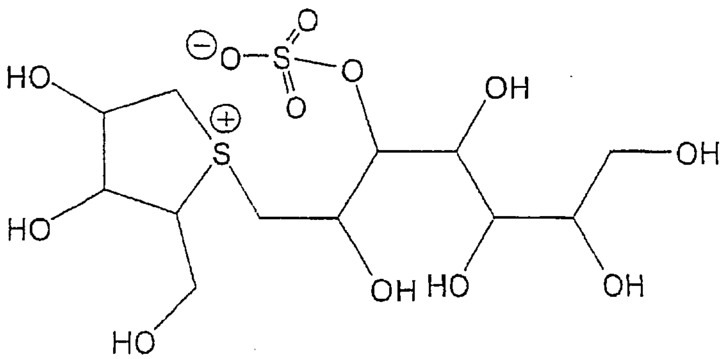

kotalanol

The object of the present invention is to find a novel substance having α-glucosidase inhibiting activity effective for prevention of diabetes and obesity, which is highly safe with low possibility of side effects in the living body and has an effective α-glucosidase inhibiting activity, from natural products having milder side effects than synthetic pharmaceutical preparations.

Disclosure of Invention

When solvent extracts from plants of the family Hippocrateaceae or the family Celastraceae, the genus Salacia, to which the above-mentioned Salacia reticulata belongs, particularly solvent extracts from their roots and stems, were examined in detail for their α-glucosidase inhibiting activity, the present inventors speculated that besides the above-mentioned Salacinol and kotalanol, a substance having a higher α-glucosidase inhibiting activity than that of Salacinol and kotalanol be contained therein. Accordingly, the present inventors made further study, and as a result, they have found a novel substance having α-glucosidase inhibiting activity.

Accordingly, the present invention provides a substance contained in a solvent extract from roots and stems of plants of the family Hippocrateaceae or the family Celastraceae, the genus Salacia, which is represented by the following formula:

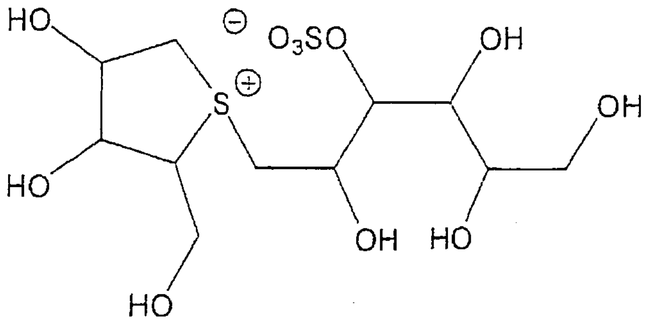

In the present invention, the novel substance having α-glucosidase inhibiting activity represented by the above formula is referred to hereinafter as "reticulanol" in order to distinguish it from the known substances (Salacinol, kotalanol etc.) having similar structures, contained in plants of the genus Salacia.
The substance "reticulanol" of the invention having α-glucosidase inhibiting activity is contained in solvent extracts from roots and stems of plants of the family Hippocrateaceae or the family Celastraceae, the genus Salacia. The plants of the genus Salacia include not only Salacia reticulata but also Salacia oblonga, Salacia chinensis, Salacia macrophylla, Salacia exculpta, Salacia prinoides, Salacia undulata etc.
The reticulanol of the present invention is contained in a solvent extract from plants of the genus Salacia. A method of preparing the solvent extract and a method of isolating and purifying reticulanol from the same are briefly described below, and the details of these methods are described in

Examples.

First, an extracting solvent which is about 10 times as large as the total weight of dried roots or stems of a plant of the genus Salacia is added to the roots or stems. The extracting solvent may be selected from water, alcohols such as methanol, and mixtures of water and alcohols or ketones such as acetone. Water is used particularly preferably as the extracting solvent.
Then, the sample is refluxed for 1 to 3 hours under heating at a temperature in the vicinity of the solvent used. After reflux, the sample is filtered while it is hot or after cooling. Then, the resulting filtrate is concentrated under reduced pressure. Depending the case, a solvent is newly added to the extraction residues, and the above procedure may be repeated further once or twice.
The solvent is distilled away from the resulting filtrate, whereby a desired solvent extract is obtained.
Thereafter, the solvent extract may be further subjected to liquid/liquid partition with an organic solvent such as hexane, ethyl acetate or n-butanol, for the purpose of purification by removing unnecessary components.
Then, the solvent extract is subjected to a conventional separation and purification method, for example column chromatography, whereby reticulanol is obtained as the novel substance of the invention having α-glycosidase inhibiting activity.
The desired reticulanol of the present invention is contained in a large amount in aqueous extracts from roots and stems of particularly Salacia reticulata, among solvents extracts from roots and stems of plants of the family Hippocrateaceae or the family Celastraceae, the genus Salacia.
In the present invention, reticulanol may be used in an isolated form or as the solvent extract containing reticulanol before separation (for example, as a reticulanol containing composition having α-glucosidase inhibiting activity).
The present invention also provides food containing, as an active ingredient, the reticulanol or the composition having α-glucosidase inhibiting activity of the present invention. Such food is effective not only in, for example, prevention and treatment of diabetes but also in prevention and amelioration of obesity.
The food of the present invention is provided more preferably in an orally ingestible form. Accordingly, the food of the present invention may if necessary contain not only the active ingredient described above, but also other known compounds used conventionally in the field of medicine or compounds necessary for forming it into powder, a solid or a liquid. Examples of such compounds include erythritol, maltitol, hydroxypropyl cellulose, kaolin, talc, and calcium carbonate.
The food of the present invention may contain the reticulanol of the present invention in such an amount that its α-glucosidase inhibiting activity can effectively function; for example, the food may contain the reticulanol in an amount of 0.0001 wt%, preferably 0.0005 to 0.01 wt%, more preferably 0.001 to 0.005 wt%, based on the total weight of the food.
The present invention is described in more detail by reference to Preparative Examples and Examples below,

but the present invention is not limited to Preparative Examples and Examples.

Examples

Preparative Example 1

Method of preparing an aqueous extract from roots and stems of Salacia reticulata

A suitable amount out of 10 L water was added to 1500 g dried roots and milled stems of Salacia reticulata which were then extracted at 100°C for 2 hours. The resulting extract was filtered, and a filtrate containing the active component was obtained. The extraction residues were subjected further twice to the above procedures with water.

The filtrates obtained by the above procedures were combined and concentrated at 40°C under reduced pressure with an aspirator of water-running type to give an aqueous extract of Salacia reticulata (202 g).

Preparative Example 2

Isolation of reticulanol

The aqueous extract (202 g) from roots and stems of Salacia reticulata, obtained in Preparative Example 1, was dissolved in 0.6 L water under warming at room temperature and then separated by centrifugation (1500 rpm, 5 minutes) into a water-soluble portion (135 g) and a water-insoluble portion (62 g). The water-soluble portion (135 g) was further fractionated into 5 fractions (referred to hereinafter as Frs. 1 to 5) by silica gel column chromatography. The conditions for separation by column chromatography and the respective fractions thus obtained are as follows: Silica gel (1300 g): solvent, $CHCl_3$ : MeOH : $H_2O$ = 60:40:10 (Fr. 1) → 55:45:10 (Fr. 2) →50:50:10 (Fr. 3) → 30:70:10 (Fr. 4) → water-containing acetone (Fr. 5).

The above Fr. 3 (17.8 g) was purified by preparative HPLC [detector, differential refractometer; column, YMC-pack Polyamide II (diameter ϕ2.0×25.0 cm); flow rate: 9.0 ml/min.; solvent: 70% aqueous acetonitrile] thereby being fractionated into 6 fractions (Fr. 3-1, Fr. 3-2, Fr. 3-3, Fr. 3-4, Fr. 3-5 and Fr. 3-6).

When a qualitative analysis of the respective fractions thus obtained was conducted by proton ($^1H$) and carbon ($^{13}C$) nuclear magnetic resonance (NMR) and FAB-MS (fast atom bombardment mass spectrometry), Frs. 3 and 2 were identified as Salacinol (yield 0.0321% based on the aqueous extract of Salacia) and Frs. 3-6 as kotalanol (yield 0.0090% based on the same extract).

Based on the following spectral data, Frs. 3-5 were found to be a novel substance reticulanol (yield 0.0142%) having the following structural formula:

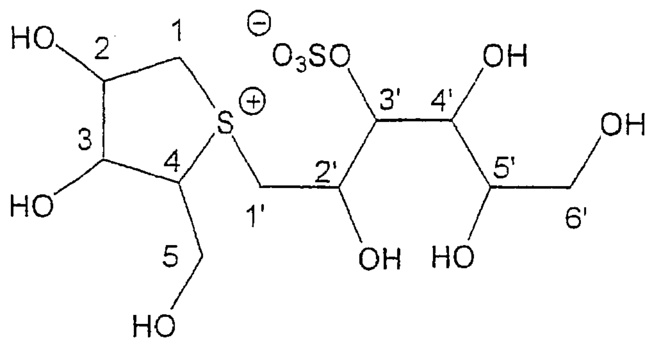

Fab-MS m/z 392.8 $(M-H)^+$

$^1H$-NMR (heavy water, 500 MHz): d 4.47 (1H, dd, J=3.5, 7.5 Hz), 4.20(1H, dd, J=3.5. 7.5Hz), 4.19-4.10 (3H, m), 3.84-3.60 (5H, m), 3.62 (2H, d, J=3.5 Hz), 3.50 (1H, dd, J=3.0, 12.0 Hz), 3.39 (1H, dd, J=6.0, 12.0 Hz).

$^{13}C$-NMR (heavy water, 126 MHz): d 79.0* (3'), 77.3* (3), 76.2 (2), 71.0 (5'), 69.5 (4), 68.3 (4), 66.0 (2'), 61.7 (6'), 58.6 (5), 49.8 (1'), 47.4 (1)

(*: Interchangeable).

Example 1: Measurement of α-glucosidase inhibiting activity

Preparation of a crude enzyme

One gram intestinal acetone powder rat (manufactured by SIGMA) was suspended in 10 mL of 0.1 M maleate buffer, pH 6.0 and then centrifuged (3,000 rpm, 4°C, 20 min.). The resulting supernatant was separated as a crude enzyme stock solution. This crude enzyme stock solution was diluted at the degree of dilution previously determined in the following control test, to prepare a crude enzyme dilution which was then used in the subsequent measurement of sucrase inhibiting activity.

Control test:

A buffer solution 50 μL and 100 μL of 74 mM aqueous sucrose solution were pipetted into each test tube and pre-heated for 3 minutes in a water bath at 37°C. A diluted crude enzyme stock solution 50 μL was added to this test tube and reacted at 37°C for 30 minutes. After the reaction was finished, 800 μL purified water was added. The mixture was further heated at 80°C for 3 minutes thereby inactivating the enzyme to terminate the reaction. The concentration of glucose in the reaction solution was quantified by using a commercial kit/mutarotase-glucose oxidase method (Glucose CII Test Wako, manufactured by Wako Pure Chemical Industries, Ltd.). As a result, it was determined that the crude enzyme stock solution be diluted 3-fold with 0.1 M maleate buffer, pH 6.0, such that the amount of glucose formed became 5 mg/dL.

Measurement of sucrase inhibiting activity of a test substance

As the test substance, the reticulanol of the present invention, and Salacinol and kotalanol for comparison, all of which were obtained in Preparative Example 2 above, were used. A solution 50 μL of the test substance in 0.1 M maleate buffer, pH 6.0 was introduced into each test tube (concentration of the test substance: 3 to 30 μg/mL), and 100 μL of 74 mM aqueous sucrose was pipetted into each test tube and pre-heated for 3 minutes in a water bath at 37°C. The above crude enzyme dilution 50 μL was added to each test tube and reacted at 37°C for 30 minutes. After the reaction was finished, 800 μL purified water was added to each test tube, and the mixture was further heated at 80°C for 3 minutes thereby inactivating the enzyme to terminate the reaction.

After the reaction was terminated, the concentration of glucose in the reaction solution was quantified by using the glucose CII Test Wako (manufactured by Wako Pure Chemical Industries, Ltd.).

Separately, the Salacinol obtained in Preparative Example 2 above was used as a comparative test substance, and reacted and quantified in the same manner as described above.

Blank test:

An aqueous sucrose solution 100 μL was mixed with 50 μL of each test substance dissolved in a maleate buffer. Purified water 800 μL was added thereto and then 50 μL crude enzyme dilution was added thereto, and immediately the mixture was heated to 80°C to inactivate the enzyme. Thereafter, the concentration of glucose in the reaction solution was measured by the same method as described above.

Calculation of $IC_{50}$ value

Sucrase inhibiting activity was calculated from the following equation:

$$\text{Sucrase inhibiting activity (\%)} = \{(Glc_0 - Glc_x)/Glc_0\} \times 100$$

wherein $Glc_x$ is a value obtained by subtracting the glucose concentration in the corresponding blank test from the concentration of glucose in the test sample, and Glco is the glucose concentration in the corresponding control.

An inhibition curve was prepared by plotting the inhibiting activity (%) calculated by the above equation on the ordinate and the concentration of each test substance (μg/mL) on the abscissa. From this inhibition curve, 50% inhibition concentration ($IC_{50}$ value) was calculated as an indicator of sucrase inhibiting activity. The results ($IC_{50}$) are shown below.

In the above experiment:

Table 1

| Test substance | $IC_{50}$ (μg/mL) |
|---|---|
| Reticulanol (the invention) | 0.38 |
| Salacinol (for comparison) | 0.81 |
| Kotalanol (for comparison) | 0.18 |

As can be seen from these results, reticulanol (the invention), Salacinol (for comparison) and kotalanol (for comparison), isolated from an aqueous extract of dry roots and milled stems of Salacia reticulata, exhibit very strong α-glucosidase inhibiting activity, and the α-glucosidase inhibiting activity of the reticulanol of the present invention is at least twice as high as the α-glucosidase inhibiting activity of Salacinol.

Pharmaceutical Preparative Example 1: Production of chewable tablets (the invention)

Table 2

| Composition | Compounding amount* (wt%) |
|---|---|
| Aqueous extract obtained in Preparative Example 1 from an aqueous extract of roots and stems of Salacia reticulata | 10.0 |
| Maltosyl cyclodextrin | 14.0 |
| Corn starch | 11.0 |
| Glucose | 42.5 |
| Gelatin | 5.0 |
| 1-Menthol | 1.0 |
| Perfume | 0.5 |
| Calcium hydrogen phosphate anhydride | 15.0 |
| Sucrose fatty ester | 1.0 |

*: the total amount of the whole composition is 100 wt%.

In the above composition, all the ingredients other than the perfume, 1-menthol and sucrose fatty ester were kneaded with one another. Then, 1-menthol and sucrose fatty ester were added thereto and further kneaded, and finally the perfume was added thereto, and the mixture was formed by extrusion granulation into granules. Then, the granules were dried at 40°C and formed by a tabletting machine into chewable tablets in an easily orally ingestible form.

The resulting chewable tablets contain the aqueous extract of Salacia reticulata in the present invention exhibiting high α-glucosidase inhibiting activity.

Example 2: Production of the drink (the invention)

Table 3

| Composition | Compounding amount |
|---|---|
| Aqueous extract obtained in Preparative Example 1 from an aqueous extract of roots and stems of Salacia reticulata | 1000 mg |
| Sodium DL-tartrate | 10 mg |
| Erythritol | 10 g |
| Citric acid | 1.2 g |
| Succinic acid | 1 ml |
| Vitamin C | 1 g |
| Perfume | 1 ml |

The above whole composition was dissolved in 800 ml distilled water, and then distilled water was added to the solution to adjust the total volume to 1000 ml. The resulting solution was sterilized through 0.22 μm sterilizing filter and charged aseptically in a volume of 100 ml into each brown bottle to produce a drink. This drink had been blended with 100 mg/bottle of the aqueous solvent extract from Salacia reticulata in the present invention, as an active ingredient effective for α-glucosidase inhibiting action.

Effect of the Invention

Reticulanol, that is, the novel substance of the invention having α-glucosidase inhibiting activity is contained in solvent extracts of natural plants, and has a thioglycosulfonyl intramolecular sulfate structure similar to that of known active ingredients (Salacinol and kotalanol). The reticulanol of the present invention is derived from a natural plant which has been used for a long period in traditional medicine in South Asia, and is thus safer in the living body than the synthetic pharmaceutical preparations.

The reticulanol of the present invention can be ingested every day in the form of, for example, food etc. compounded therewith. Owing to the α-glucosidase inhibiting activity of the reticulanol, such food can suppress postprandial hyper-blood sugar, and is thus not only effective in prevention of diabetes but also expectable for application to amelioration of the symptom (that is, treatment) of patients with diabetes.

The reticulanol of the present invention exhibits an excellent α-glucosidase inhibiting activity, and is thus considered highly possible for it to become a lead chemical in pharmaceutical preparations in the future. According to the present invention, reticulanol became one of compounds evidencing a blood sugar level-ameliorating action possessed potentially by plants of the genus Salacia.

**Claims**

**1.** A substance having α-glucosidase inhibiting activity, represented by the following formula:

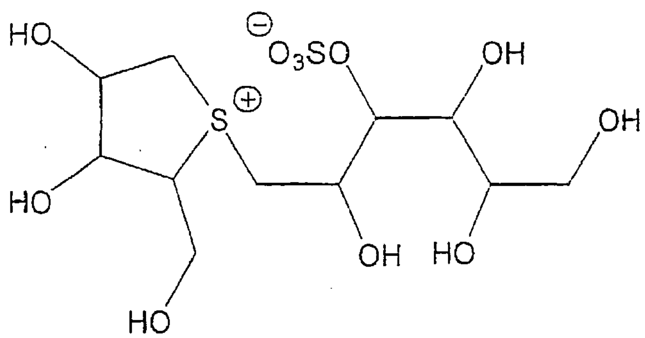

**2.** The substance having α-glucosidase inhibiting activity according to claim 1, which is contained in a solvent extract of roots and stems of a plant of the family Hippocrateaceae or in the family Celastraceae, the genus Salacia.

**3.** The substance having α-glucosidase inhibiting activity according to claim 2, wherein the plant is Salacia reticulata.

**4.** A composition having α-glucosidase inhibiting activity, which comprises the substance having α-glucosidase inhibiting activity according to claim 1.

**5.** Food comprising the substance having α-glucosidase inhibiting activity according to claim 1.

**6.** Food comprising the composition having α-glucosidase inhibiting activity according to claim 4.

**INTERNATIONAL SEARCH REPORT**

International application No.
PCT/JP2004/005865

A. CLASSIFICATION OF SUBJECT MATTER
Int.Cl[7] C07D333/46, A61K31/381, 35/78, A61P3/04, 3/10, 43/00, A23L1/30, C12N9/99

According to International Patent Classification (IPC) or to both national classification and IPC

B. FIELDS SEARCHED

Minimum documentation searched (classification system followed by classification symbols)
Int.Cl[7] C07D333/46, A61K31/381, 35/78, A61P3/04, 3/10, 43/00, A23L1/30, C12N9/99

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2004 |
| Kokai Jitsuyo Shinan Koho | 1971-2004 | Toroku Jitsuyo Shinan Koho | 1994-2004 |

Electronic data base consulted during the international search (name of data base and, where practicable, search terms used)
CAplus(STN), REGISTRY(STN), MEDLINE(STN), EMBASE(STN), BIOSIS(STN), BIOTECHABS(STN), WPI(DIALOG), JSTPLUS(JOIS), JMEDPLUS(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y | Masayuki YOSHIKAWA et al., "Salacia-Zoku Shokubutsu no Polyphenol Seibun: α-Glucosidase Oyobi Aldose Reductase Sogai Kassei Seibun", Mangiferin, no Teiryo Bunseki, Journal of the Pharmaceutical Society of Japan, 2001, 121(5), pages 371 to 378, full text | 2,3<br>1,4-6 |
| X<br>Y | YOSHIKAWA, M. et al., Kotalanol, a Potent α-Glucosidase Inhibitor with Thiosugar Sulfonium Sulfate Structure, from Antidiabetic Ayurvedic Medicine Salacia reticulata., Chem. Pharm.Bull., 1998, 46(8), pages 1339 to 1340 | 2,3<br>1,4-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 August, 2004 (12.08.04) | 31 August, 2004 (31.08.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/005865

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | YOSHIKAWA, M. et al., SALACINOL, POTENT ANTIDIABETIC PRINCIPLE WITH UNIQUE THIOSUGAR SULFONIUM SULFATE STRUCTURE FROM THE AYURVEDIC TRADICIONAL MEDICINE Salacia reticulata IN SRI LANKA AND INDIA., Tetrahedron Lett., 1997, 38(48), pages 8367 to 8370 | 2,3<br>1,4-6 |
| X<br>Y | MATSUDA, H. et al., Antidiabetic Principles of Natural Medicines., IV., Aldose Reductase and α-Glucosidase inhibitors from the Roots of Salacia oblonga Wall. (Celastraceae): Structure of a New Friendelane-Type Triterpene, Kotalagenin 16-Acetate., Chem.Pharm.Bull., 1999, 47(12), pages 1725 to 1729 | 2,3<br>1,4-6 |
| X<br>Y | JP 2000-86653 A (Rankaayurubetikkuhabu Yakuhin Kabushiki Kaisha),<br>28 March, 2000 (28.03.00),<br>Full text<br>(Family: none) | 2,3<br>1,4-6 |
| X<br>Y | JP 11-29472 A (Research Institute For Production Development),<br>02 February, 1999 (02.02.99),<br>Full text<br>& US 2002/0041904 A1 & US 6376682 B1 | 2,3<br>1,4-6 |
| X<br>Y | Osami KAJIMOTO et al., "Salacia reticulata Mizu Chushutsubutsu ni Okeru Kyokaigata Oyobi Keisho 2-gata Tonyobyo Shorei ni Taisuru Rinsho Koka", Journal of Japanese Society of Nutrition, and Food Science, 2000, 53(5), pages 199 to 205, full text | 2,3<br>5,6 |
| X<br>Y | JP 2001-103928 A (Fancl Corp.),<br>17 April, 2001 (17.04.01),<br>Full text<br>(Family: none) | 2,3<br>5,6 |
| Y | WO 01/49674 A2 (SIMON FRASER UNIVERSITY),<br>12 July, 2001 (12.07.01),<br>Full text<br>& AU 200126591 A & US 6455573 B1<br>& EP 1248779 A2 & CN 1404476 A<br>& JP 2003-519220 A & US 2003/0191104 A1 | 1-6 |
| A | JP 2002-104979 A (Nippon Kefia Kabushiki Kaisha),<br>10 April, 2002 (10.04.02),<br>(Family: none) | 5,6 |

**INTERNATIONAL SEARCH REPORT**

International application No.
PCT/JP2004/005865

C (Continuation). DOCUMENTS CONSIDERED TO BE RELEVANT

| Category* | Citation of document, with indication, where appropriate, of the relevant passages | Relevant to claim No. |
|---|---|---|
| P,X<br>P,Y | Masayuki YOSHIKAWA et al., "Thai-san Salacia chinesis no Seibutsu Kassei: α-Glucosidase Sogai Kassei o Shihyo to shita Hinshitsu Hyoka", Journal of the Pharmaceutical Society of Japan, 2003, 123(10), pages 871 to 880 | 2,3<br>1,4-6 |
| P,X<br>P,Y | JP 2003-171299 A (Bio Venture Bank Co., Ltd.), 17 June, 2003 (17.06.03), (Family: none) | 2,3<br>5,6 |

Form PCT/ISA/210 (continuation of second sheet) (January 2004)